# EUROPEAN PATENT SPECIFICATION

(11) **EP 1 578 392 B1**
(45) Date of publication and mention of the grant of the patent: **04.08.2010**
(21) Application number: 03799508.1
(22) Date of filing: 24.12.2003
(51) Int. Cl.: A61K 8/04, A61K 8/06, A61K 8/34, A61K 8/365, A61K 8/89, A61Q 19/00

(54) **SPRAYABLE OIL-LIKE FORMULATIONS**
SPRÜHBARE ÖLARTIGE FORMULIERUNGEN
FORMULATIONS DE TYPE HUILE POUVANT ETRE VAPORISEES

(30) Priority: 31.12.2002 EP 02080680
(43) Date of publication of application: 28.09.2005
(73) Proprietor: Johnson & Johnson GmbH, 40474 Düsseldorf (DE)
(72) Inventor: STORK, Anja, 53113 Bonn (DE); ARIF, Ambareen, Ile Le Croix, 76000 Rouen (FR); MUTTI, Bruna, Residence St. Huber, 76240 Mesnil Esnard (FR)
(74) Representative: Metten, Karl-Heinz
(86) International application number: PCT/EP2003/014905
(87) International publication number: WO 2004/058212

(56) References cited:
- EP-A- 0 930 066
- EP-A2- 0 829 253
- WO-A-02/100922
- JP-A- 2002 205 911
- N. GARTI ET AL.: "Transparent macroemulsions for cosmetic applications" INTERNATIONAL JOURNAL OF COSMETIC SCIENCE, vol. 8, no. 1, 1986, pages 1-8, XP008017221

## Description

### Field of the invention

This invention relates to transparent, sprayable cosmetic oil-in-water formulations that provide good moisturization.

### Background of the Invention

A clear and transparent appearance of skin care or cosmetic products has become an important feature as the consumer associates it with attributes such as pureness, mildness, cleanliness and freshness. Another benefit of a clear appearance, in combination with a transparent packaging, is that the consumer is readily able to view and inspect the product.

One particular product category, where clearness is an important attribute, are cosmetic oils. These are very traditional and well-established skin-care products for adults as well as for babies. They have been used for many years and have become attractive due to their excellent skin moisturizing and protecting properties that are superior to ordinary creams and lotions.

However, due to their low consistency, oils can be messy to apply, leaving a long lasting greasy skin feel, and in particular are difficult to apply on dry skin.

Formulations have been developed with the aim to avoid an unpleasant skin feel. For example EP-A-930 066 describes formulations comprising a mixture of a silicone wax, a silicone fluid and two long chain lactate molecules that have a reduced tacky skin feel and are suitable for use in creams, gels, lotions and salves. WO-02/100922 features branched organosilicone compounds useful as a component in personal care compositions having improved sensory feel and leaving a smooth silky feeling in the skin.

Garti et al. ("Transparent macro-emulsions for cosmetic applications" International Journal of Cosmetic Science, vol. 8, no. 1, 1986, pages 1 - 8, X008017221) is about a method for the preparation of transparent emulsions for cosmetic applications. The continuous phase in the oil-in-water emulsions consists of both water and several polyols in order to equalize the refractive indices of the two phases. Combinations of triethanol amine oleate together with sorbitan esters and ethoxylated sorbitan esters are described to improve the emulsion stability. Further, it was found that the lower the refractive index of the oil phase, the lower the polyol concentration which will be required.

In EP 0 829 253 A2 a method for the preparation of a water-base toiletry preparation is disclosed which comprises the steps of (a) preparing an aqueous dispersion of spherical particles of a cured silicone rubber having an average particle diameter in the range from 0.1 to 100 µm dispersed in an aqueous medium containing a surface active agent, the amount of the cured silicone rubber particles being in the range from 0.1 to 20 % by weight and the balance being water, each based on the amount of the aqueous dispersion; and (b) admixing a base mixture of a water-base toiletry preparation with the aqueous dispersion of the cured silicone rubber particles prepared in step (a) in such a proportion that the content of the cured silicone rubber particles in the toiletry preparation is in the range from 0.1 to 30 % by weight based on the total amount of the non-volatile matters in the base mixture. By employing the aforementioned method preparations shall be obtained which exhibit excellent touch feeling after treatment even when the toiletry preparation is a water-base composition.

In JP 2002/205911 A an aqueous cosmetic preparation for the skin is described which contains 2 to 30 weight-% of a polyhydric alcohol, 0.05 to 15 weight-% of a thickener and an aqueous emulsion containing cross-linked silicone particles having a mean particle diameter of 0.05 to 100 µm in an oil drop with a mean particle diameter of 0.01 to 500 µm. The aqueous cosmetic thus obtained has only a slight feeling of tackiness, a light spread during use and excellent refreshing feeling as well as excellent storage stability.

Sprays are attractive as a vehicle to apply liquid compositions for topical use. However oil sprays are hampered with toxicological problems due to inhalation of finely dispersed oil droplets and more specifically are not safe enough for baby usage.

There is a need for sprayable oily or oil-like formulations that are transparent and have the same beneficial properties as the oils that are currently used, but lack the oily/greasy skin feel of traditional oil formulations. The oil-like formulations of this invention having the composition set forth hereinafter, have been found to meet this and some other needs.

### Summary of the invention

The present invention concerns a clear sprayable oil-in-water emulsion according to claim 1.

Suitable silicone waxes are condensation products of alkenyl substituted polysiloxanes and polysiloxanes with silane functionalities.

Suitable oils may be selected from silicone oils, natural oils, fatty acid esters, ether and mono-, di- and triglycerides, cyclic, branched or linear hydrocarbons, linear or branched fatty alcohols (Guerbet alcohols), and mixtures thereof. Of particular interest are silicone oils.

Preferably the polyol is a polyhydroxy alkane or -cycloalkane.

Preferably the emulsifier is an ethoxylated fatty alcohol.

### Detailed description of the invention

As used herein the term 'clear' means that the emulsion is transparent or essentially transparent when present in a typical consumer product, allowing ready viewing of objects behind it (when packed in a transparent container or packaging). In particular, the formulations of the present invention have a transparency (T % as measure unit) of T ≥95%. The transparency may be measured with a UV/VIS double ray spectrometer at a wavelength of 800 nm.

The term 'sprayable' means that the composition can be applied by a standard spraying device used in consumer products. In particular, sprayable emulsions have a viscosity of < 100mPas measured with a plate/cone rotation rheometer at a constant shear rate of 500 s⁻¹. Preferably, sprayable emulsions in accordance with the present invention have a viscosity from 1 - 30 mPas, in particular from 1-15 mPas, or from 1- 15 mPas at a shear rate of 500 s⁻¹.

### Silicone Waxes

The silicone waxes for use in the emulsions according to the invention are copolymeric condensation products of alkenyl substituted polysiloxanes and polysiloxanes with silane (Si-H) functionalities. Particular alkenyl substituted polysiloxanes are those wherein the alkenyl functions are end groups and polysiloxanes with silane (Si-H) functionalities are those wherein the silane groups are end groups.

The alkenyl substituted polysiloxanes and polysiloxanes with silane functionality are linear but in some instances may have limited branching, for example less than about 2 mole % of the siloxane units, in which case the polysiloxanes are 'substantially linear'.

Preferably, the copolymeric silicone waxes are obtained by condensation of about 10- 40 parts, in particular of about 20-35 parts, more in particular from about 25 to about 35 parts of alkenyl substituted polysiloxane with about 0.25-5 parts, in particular of about 0.5-2 parts, more in particular from about 0.65 to about 1.5 parts of polysiloxanes with silane (Si-H) functionalities.

Preferred alkenyl substituted polysiloxanes are those wherein the alkenyl group is vinyl, allyl, hexenyl or cyclohexenyl, more preferably vinyl.

In specific embodiments of this invention, the silicone wax is a condensation product of divinyldimethicone and a silane-terminated dimethicone.

These silicone waxes can be prepared by condensing the alkenyl substituted polysiloxanes with the polysiloxanes with silane functionalities in the presence of a suitable catalyst, for example a suitable metal catalyst such as Pt. The reaction can be done in the presence of an appropriate emulsifier, whereupon after addition of water an emulsion of the silicone wax in water is obtained.

Particular silicone waxes for use in the emulsions of the present invention are those described in US-6,013, 682, more in particular the divinyldimethicone/dimethicone copolymer specifically described in the examples of said reference. Silicone-in-water emulsions of these waxes as well as their preparation are also described in this reference.

Preferred for use in the present invention are silicone waxes directly prepared in an emulsion as described in US-6,013, 682, in combination with a suitable non-ionic

emulsifier, in particular with an ethoxylated fatty alcohol. Of interest are silicone wax emulsions having a particle size in the range from about 0.3 to about 100 micrometers and a viscosity that is in the range of about 1 mm²/sec at 25 °C to about 10⁸ mm²/sec at 25 °C. Of specific interest are wax emulsions of a particle size, which is in the range of about 1 to about 100 micrometers and a viscosity, which is the range of about 10⁶ to about 10⁸ mm²/sec.

These emulsified silicone waxes can be readily used to prepare the emulsions according to the present invention.

Preferably, the silicone wax or waxes are present in the emulsions of the invention in an amount, which is in the range of 0.1 - 30%, preferably in the range of 2 - 18 %, more preferably from 5 - 12 % (w/w, relative to the total weight of the emulsion).

### Oils

The emulsions of the present invention may contain suitable oils which are skin-compatible components or component mixtures that are non water-mixable and which may, for example, be silicone oils, natural oils, fatty acid esters, mono-, di- or triglycerides, or other oils, or mixtures thereof. Preferably, the oils are liquid at ambient temperature, in particular are liquid at 20 °C or at 25 °C. They can contain certain amounts of solid lipid components (e.g. fats) as long as the complete oil mixture is liquid at ambient temperature or at the temperatures mentioned above.

Of particular interest are silicone oils such as, for example cyclic silicones, dialkyl- or alkylarylsiloxanes, e.g., cyclomethicone, dimethyl polysiloxane and methylphenyl polysiloxane, as well as the alkoxylated and quatemized analogs thereof. Appropriate non-volatile silicone oils are e.g. polyalkylsiloxanes, polyalkylarylsiloxanes and polyethersiloxane-copolymers. A particularly suitable silicone oil can be a dimethylpolysiloxane having trimethylsiloxy groups at both molecular terminals, a methylphenylpolysiloxane having trimethylsiloxy groups at both molecular terminals, a copolymer of methylphenylsiloxane and dimethylsiloxane having trimethylsiloxy groups at both molecular terminals, a cyclic dimethylsiloxane, or a cyclic methylphenylsiloxane.

Preferred silicone oils comprise cyclic dimethylsilicones, i.e. the cyclomethicones e.g. tetracyclomethicone, pentacyclomethicone, and linear dimethylsilicones, i.e. the dimethicones, including any mixture of these.

Other oils, which can be incorporated comprise natural oils or fats, or natural oil derivatives, in particular of vegetable origin. Examples are almond oil, soybean oil, sunflower oil, safflower oil, corn oil, canola oil, borage oil, evening primrose oil, grapeseed oil, wheat germ oil, avocado oil, jojoba oil, kernel oil, sesame oil, linseed oil, palm oil, olive oil, macadamia oil, castor oil, rapeseed oil, coconut oil, and turnip seed oil. Also included are oil components isolated from natural oils, more specifically from the natural oils mentioned above.

The major components of the natural oils are mono-, di and in particular triglycerides. These glycerides themselves can be used as oils in the emulsions of the invention. The mono-, di or triglycerides may be derived from saturated or unsaturated, linear or branch chained, substituted or unsubstituted fatty acids or fatty acid mixtures. Particular mono-, di- or triglycerides are mono-, di- or tri-C₆₋₂₄ fatty acid glycerides, specifically mono-, di-or tri-C₈₋₂₂ fatty acid glycerides, more specifically mono-, di-or tri-C₁₂₋₂₀ fatty acid glycerides, for example glyceryl monostearate, distearate or tristearate. Mixtures of mono-, di- and triglycerides can be derived from fractions of fatty acids. An example of the latter C₁₂₋₁₈ fatty acid mono-, di- and triglycerides mixtures.

Of interest are the triglycerides that can be isolated from natural oils or can be prepared chemically, e.g. glycerine tristearate, glycerine tribehenate, glycerine tripalmitate, glycerine trilaurate, glycerine trioleate, glycerine trimyristate. The fatty acids in the triglycerides can be the same or different, the latter being mixed glyceride esters. Mono- or diglycerides can be used as such but are usually used in mixture with triglycerides. Examples are glyceryl monostearate and glyceryl distearate.

The formulations may also comprise alkyl esters of fatty acids. The alkyl groups in said esters may have from 1 to 30 carbon atoms, in particular from 6 to 24 carbon atoms more in particular from 12 to 22 carbon atoms. The alkyl groups preferably are derived from fatty alcohols as well as from fatty alcohol mixtures, in particular from linear or branched, saturated or unsaturated fatty alcohols such as C₆-C₃₀-fatty alcohols, in particular C₈₋₂₄ fatty alcohols, more in particular C₁₂₋₂₀ fatty alcohols. The fatty acids in said alkyl esters may be linear or branched, saturated or unsaturated fatty acids, in particular are C₆₋₃₀ fatty acids, more in particular C₁₂₋₂₄ fatty acids, further in particular C₁₂₋₂₀ fatty acids, or C₁₆₋₁₈ fatty acids. Preferred are esters from C₆-C₂₂-fatty acids with C₆-C₂₂-fatty alcohols.

Examples of oil components of the ester type are the following: decyl oleate, coco caprylate/-caprate, hexyl laurate, myristyl isostearate, myristyl oleate, cetyl isostearate, cetyl oleate, stearyl isostearate, isostearyl myristate, isostearyl palmitate, isostearyl stearate, isostearyl isostearate, isostearyl oleate, isostearyl oleate, oleyl myristate, oleyl isostearate, oleyl oleate, oleyl erucate, behenyl isostearate, erucyl isostearate, erucyl oleate. Other oil components are esters from linear and/or branched fatty acids with multifunctional alcohols (e.g. propylene glycol, dimerdiol oder trimertriol) and/or Guerbet alcohols, as well esters from C₆-C₂₂-fatty alcohols and/or Guerbet alcohols with aromatic carboxylic acids, in particular benzoic acids, esters from C₂-C₁₂-dicarboxylic acids with linear or branched alcohols having 1 to 22 carbon atoms (e.g. dioctyl maleates).

Other oil components that may be used are the fatty alcohols. These can be saturated or unsaturated, straight or branch chained fatty alcohols, including mixtures thereof. Examples are C₁₂-C₅₀-fatty alcohols, in particular the C₁₂-C₂₄-fatty alcohols, more in particular the C₁₆-C₂₂-fatty alcohols e.g. myristyl alcohol, 1-pentadecanol, cetylalcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol, myricyl alcohol, lauryl alcohol, capryl alcohol, caprinyl alcohol, cetyl alcohol, palmoleyl alcohol, isostearyl alcohol, oleyl alcohol, elaidyl alcohol, petroselinyl alcohol, arachidyl alcohol, gadoleyl alcohol, erucyl alcohol, including mixtures thereof such as cetearyl alcohol, C_{12/13} fatty alcohol.

Still other oils are the C₁₄-C₄₀-fatty acids, including mixtures thereof. Of particular interest are the C₁₆-C₃₀-fatty acids. These comprise, for example, myristic-, pentadecanoic-, palmitic-, margaric-, stearic-, nonadecanoic-, arachic-, behenic-, lignoceric-, cerotic-, melissic-, erucaic-, elaeostearic-, oleic-, lonolenic-, lauric acid as well as substituted fatty acids, e.g. hydroxy-substituted fatty acids such as, for example, 12-hydroxystearic acid, and the amides or monoethanolamides of these fatty acids.

Further oils are the dialkyl(ene) ethers which can be symmetric or asymmetric, straight or branch chained, saturated or unsaturated. Of interest are C₆-C₃₀-dialkylethers, in particular C₆-C₂₄-dialkylethers, more in particular C₆-C₂₀-dialkylethers, e.g. distearylether, dibehenylether, di-n-octylether, di-(2-ethylhexyl)-ether, laurylmethylether, octylbutylether, didodecylether. These ethers can be obtained from the appropriate fatty alcohols, in particular the fatty alcohols mentioned above, following art-known procedures.

The dialkyl(ene) carbonates that can be used are symmetric or asymmetric, straight or branch chained, saturated or unsaturated. Of interest are linear or branch chained, saturated or unsaturated C₁₄-C₃₀-dialkyl(ene) carbonates, in particular the carbonates derived from the fatty acids mentioned above. Of interest are C₁₆-C₂₄-dialkyl carbonates and a particular subgroup are the saturated linear C₁₆-C₂₂-dialkyl carbonates, e.g. distearyl carbonate, dihexyl-, dioctyl-, di-(2-ethylhexyl)- or dioleylcarbonate.

Dicarboxylic acids that can be used are, for example, C₉-C₃₄-dicarbonic acids. These comprise, for example, octadecanedioic acid, tetratridecanedioic acid, etc. Of particular interest are the azelainic acids, which are C₉-dicarboxylic acids.

Other oil components are hydroxy fatty alcohols which are saturated or unsaturated, straight chain or branched. Of interest are C₁₂-C₃₀-hydroxy fatty alcohols, wherein the position of the hydroxy-substituent depends upon the synthesis route and the starting materials that have been used. Included are, for example, 1,10-decanediol, 1,2-hexadecanediol, 12-hydroxystearyl alcohol or hydroxy-Guerbet alcohols.

Further oil components, which can be used comprise, mineral and paraffin oils and synthetic oils, either aliphatic or aromatic, as well as mixtures thereof. Examples are the Guerbet alcohols based on fatty alcohols having 6 to 18, in particular 8 to 10 carbon atoms, or hydrocarbons, for example, squalane, squalene, paraffine oils, isohexadecane, isoeicosane, polydecene, as well as dialkylcyclohexanes.

The total amount of oils or oil components (excluding the silicone waxes) in the emulsions according to the invention can vary but generally is in the range of 0.1% - 15%, preferably 0.2% - 7%, more preferably 0.5% - 2.5% (w/w, relative to the total weight of the emulsion).

The total amount of the oil components in the emulsions of the invention, i.e. including all lipid components such as the silicone waxes and oils mentioned herein, may vary but generally is in the range of 2 - 45 %, preferably in the range of 4 - 25%, more preferably of 5 - 15% (w/w, relative to the total weight of the emulsion).

The aqueous phase contains one or more polyols or hydroxy acids or their salts. The polyols in particular are polyhydroxy alkanes or cycloalkanes. Examples of such polyols are lower alkylene glycols such as ethylene, propylene, butylenes, pentylene, and hexylene glycol; polyols such as glycerine, sorbitol, cyclohexanediol and the like. Also hydroxy acids and their salts can be used, e.g. glycolic, lactic, citric acids and the salts thereof. Salts comprise, for example, alkali metal salts, in particular potassium and sodium, ammonium or substituted ammonium salts. Of particular interest are propylene glycol and glycerine.

Polyols are added to adjust the refractive index of the aqueous phase to make it as close as possible to that of the oil phase. The refractive indices of both phases should be similar and in particular should not differ more than about 0.003, in particular about 0.002 preferably not more than 0.0012.

The quantity of the polyol that is added therefore is selected such that the refractive index of the aqueous phase meets these criteria.

The total amount of the polyols in the emulsions of the invention is in the range of from 35 % - 60%, preferably in the range of from 35 % - 50 %, more preferably of from 35 % - 45 % (w/w, relative to the total weight of the emulsion).

The aqueous or the oil phase in the emulsions of the invention may contain further components customarily used in skin-care products, e.g. certain active ingredients, perfumes, emollients and the like.

The aqueous phase in the emulsions of the invention may be present in varying amounts, but usually this phase is present in amounts which are in the range of from 50 - 98 %, preferably in the range of from 70 - 96 %, more preferably of from 85 - 95 % (w/w, relative to the total weight of the emulsion).

The compositions according to the invention further contain a suitable emulsifier. Preferably the emulsifier is selected from ethoxylated or propyloxylated fatty alcohols. These products can be obtained by an addition reaction of the appropriate amount of ethylene or propylene oxide and a fatty alcohol, in particular the fatty alcohols mentioned above. The fatty alcohols from which these products are derived can be saturated or unsaturated, straight or branch chained fatty alcohols. The fatty alcohols in particular are derived from natural fats, oils or waxes.

In one embodiment, the ethoxylated or propyloxylated fatty alcohols are derived from C₁₂-C₅₀-fatty alcohols, in particular from C₁₂-C₂₄-fatty alcohols, more in particular from C₁₆-C₂₂-fatty alcohols, or from C₈₋₂₄ fatty alcohols, or from C₈₋₁₆-fatty alcohols, including mixtures thereof. The ethoxylated or propyloxylated fatty alcohols may contain from 1 to 50, in particular 3 to 40, more in particular 5 to 30 ethoxy or propoxy units. In certain embodiments, the ethoxylated or propoxylated fatty alcohols contain from 3 to 10 ethoxy or propoxy units while in other embodiments said alcohols contain from 20 to 25 ethoxy or propoxy units. In certain other embodiments mixtures of ethoxylated alcohols of both groups are used. Preferred are the ethoxylated fatty alcohols. Of further interest are block polymers of the above mentioned PEG /PPG fatty alcohols.

Examples are ethoxylated or propoxylated myristyl alcohol, 1-pentadecanol, cetylalcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol, myricyl alcohol, lauryl alcohol, capryl alcohol, caprinyl alcohol, cetyl alcohol, palmoleyl alcohol, isostearyl alcohol, oleyl alcohol, elaidyl alcohol, petroselinyl alcohol, arachidyl alcohol, gadoleyl alcohol, erucyl alcohol, including mixtures thereof such as cetearyl alcohol, C_{12/13} fatty alcohol, as well as Guerbet alcohols.

Mixtures of fatty alcohols can evidently also be used, including fatty alcohol fractions obtained from the reduction of the corresponding fatty acid fractions derived from naturally occurring oils or fats such as, for example, almond oil, soybean oil, sunflower oil, safflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, coconut oil, and turnip seed oil.

Of interest as emulsifier are the ethoxylated or propoxylated C₈₋₂₄-fatty alcohols, preferably the ethoxylated C₈₋₁₆-fatty alcohols, having from 3 to 30 ethoxy units. Of particular interest are the ethoxylated or propoxylated fatty alcohols that contain from 3 to 10 ethoxy or propoxy units, or the same alcohols that contain from 20 to 25 ethoxy or propoxy units. In certain other embodiments mixtures of ethoxylated or propoxylated alcohols of both groups are used in combination.

Preferred emulsifiers are ethoxylated C_{12/13} alcohols (Pareth) and ethoxylated lauryl alcohol (Laureth), in particular having the level of ethoxylation mentioned above.

The total amount of the emulsifier in the emulsions of the invention may vary but generally is in the range of from 0.05 % - 10 %, preferably in the range of from 0.1 % - 5 %, more preferably of.from 0.3 % - 2 % (w/w, relative to the total weight of the emulsion).

The pH of the oil-like emulsions of this invention is selected such that they are skin-compatible. In general it is contemplated that a good skin-compatible pH is in the range of pH 4 to 8, preferably in the range of pH 5 to 6. A particularly attractive pH is pH 5.5 which is kept more or less stable, e.g. within a range of pH +/- 0.2.

To stabilize the pH, suitable buffering agents may be added such as citric acid, lactic acid or appropriate salts thereof.

The emulsions according to the present invention may contain further ingredients such as preservatives, active ingredients, perfumes and the like.

The refractive index of the complete formulation may vary but in specific embodiments of this invention the refractive index of the emulsions is about 1.3996 to about 1.4216.

The emulsions of the invention are transparent and liquid, and are oil-resembling formulations having the appearance and viscosity of a traditional oil formulation. The inventive emulsions are sprayable and have the same or even improved beneficial properties compared to traditional oil formulations, but lack their oily/greasy skin feel. The inventive emulsions have a light and soft skin feel and moreover have good moisturizing properties comparable to traditional oils and superior to the more traditional oil-in-water / water-in-oil formulations emulsions present in lotions and creams. They are devoid of the toxicological problems encountered with sprays based on traditional oils and are safe for baby usage.

### Examples

### Example 1

| **INCI name** | **Weight [%]** | **Weight [g]** |
|---|---|---|
| Divinyldimethicone/Dimethicone Copolymer, C12-C13 Pareth-3/C12-C13 Pareth-23 | 6.80 | 307.02 |
| Polysorbate 20 | 0.50 | 22.575 |
| Propylene Glycol | 4.00 | 180.60 |
| Sodium Lactate 50% | 10.00 | 451.50 |
| Water, demineralized | 36.0674 | 1628.44311 |
| Glycerine 99,5% | 41.499 | 1873.67985 |
| Citric Acid | 0.1666 | 7.52199 |
| Methylparaben | 0.20 | 9.03 |
| Perfume | 0.017 | 0.76755 |
| Sodium Chloride | 0.75 | 33.8625 |
| | | |
| | 100.00 | .4515.00 |

Divinyldimethicone/Dimethicone Copolymer is available from Dow Corning (Dow Corning HMW 2220^{™} non-ionic emulsion) and is described in US-6,013,682.

The process:
1) Premix fragrance and polysorbate 20.
2) Use approximately 2% of the water quantity specified to make citric acid solution.
3) Mix together propylene glycol, sodium lactate, glycerin, Nipagin M Sodium and remaining water.
4) Add fragrance premix.
5) Add citric acid solution.
6) Measure pH - as a guideline pH 5.4-5.5
7) In a separate beaker, premix DC HMW and Cyclopentasiloxane (only for example 2).
8) Add the water mix to the above slowly.

Measure refractive index, if necessary adjust either with water or glycerin in order to match the refractive index and obtain a transparent product.

### Example 2

| **INCI name** | **Weight [%]** |
|---|---|
| Divinyldimethicone/Dimethicone Copolymer, C12-C13 Pareth-3/C12-C13 Pareth-23 | 6.80 |
| Cyclopentasiloxane | 1.00 |
| Propylene Glycol | 4.00 |
| Sodium Lactate 50% | 10.00 |
| Water, demineralized | 43.19 |
| Glycerine 99.5% | 35.01 |
| Total | 100.00 |

Divinyldimethicone/Dimethicone isDow Corning HMW 2220^{™} non-ionic emulsion.

## Claims

1. A clear sprayable oil-in-water emulsion wherein the oily component comprises one or more silicone waxes, which are condensation products of alkenyl substituted polysiloxanes and polysiloxanes with silane functionalities, in admixture with one or more silicone oils; and optionally one or more suitable oils selected from natural oils, fatty acid esters, mono-, di- and triglycerides, cyclic, branched or linear hydrocarbons, linear or branched fatty alcohols (Guerbet alcohols), and mixtures thereof, and the aqueous phase comprises one or more polyols, wherein the emulsion further comprises an emulsifier, and wherein the polyol is present in the oil-in-water emulsion in an amount of from 35 to 60 weight-%.

2. The oil-in-water emulsion according to claim 1 wherein the polyol is present in the oil-in-water emulsion in an amount of from 35 to 50 weight-%.

3. The oil-in-water emulsion according to claim 1 wherein the oily component comprises:
(a) from 5 - 12 % of one or more silicone waxes;
(b) from 0.5 - 2.5 % of one or more silicone oils; and
(c) optionally one ore more suitable oils, and
wherein the aqueous phase comprises one or more polyols and wherein the emulsion further comprises from 0.3 - 2 % of an emulsifier.

4. The emulsion according to claim 1 or 3 wherein the polyol is a polyhydroxy alkane or - cycloalkane.

5. The emulsion according to claim 4 wherein the polyol is glycerine.

6. The emulsion according to claim 1 or 3 wherein the emulsifier is an ethoxylated fatty alcohol.

7. The emulsion according to claim 6 wherein the emulsifier is selected from ethoxylated C₈₋₁₆ fatty alcohols.

## Patentansprüche

1. Klare sprühbare Öl-in-Wasser-Emulsion, wobei die Ölkomponente umfasst: ein oder mehrere Siliconwachse, die Kondensationsprodukte alkenylsubstituierter Polysiloxane und von Polysiloxanen mit Silanfunktionalitäten sind, im Gemisch mit einem oder mehreren Siliconölen, und optional ein oder mehrere geeignete Öle, ausgewählt aus natürlichen Ölen, Fettsäureestern, Mono-, Di- und Triglyceriden, cyclischen, verzweigten oder linearen Kohlenwasserstoffen, linearen oder verzweigten Fettalkoholen (Guerbet-Alkohole) und Gemischen derselben, und die wässrige Phase ein oder mehrere Polyole umfasst, wobei die Emulsion ferner einen Emulgator umfasst und wobei das Polyol in der Öl-in-Wasser-Emulsion in einer Menge von 35 bis 60 Gew. % vorhanden ist.

2. Öl-in-Wasser-Emulsion nach Anspruch 1, **dadurch gekennzeichnet, dass** das Polyol in der Öl-in-Wasser-Emulsion in einer Menge von 35 bis 50 Gew.-% vorhanden ist.

3. Öl-in-Wasser-Emulsion nach Anspruch 1, **dadurch gekennzeichnet, dass** die Ölkomponente umfasst:
(a) 5-12 % eines oder mehrerer Siliconwachse,
(b) 0,5-2,5 % eines oder mehrerer Siliconöle, und
(c) optional ein oder mehrere geeignete Öle, und
wobei die wässrige Phase ein oder mehrere Polyole umfasst und wobei die Emulsion ferner 0,3-2 % eines Emulgators umfasst.

4. Emulsion nach Anspruch 1 oder 3, **dadurch gekennzeichnet, dass** das Polyol ein Polyhydroxyalkan oder -cycloalkan ist.

5. Emulsion nach Anspruch 4, **dadurch gekennzeichnet, dass** das Polyol Glycerin ist.

6. Emulsion nach Anspruch 1 oder 3, **dadurch gekennzeichnet, dass** der Emulgator ein ethoxylierter Fettalkohol ist.

7. Emulsion nach Anspruch 6, **dadurch gekennzeichnet, dass** der Emulgator aus ethoxylierten C₈₋₁₆-Fettalkoholen ausgewählt ist.

## Revendications

1. Emulsion d'huile dans l'eau pulvérisable transparente dans laquelle le composant huileux comprend une ou plusieurs cires de silicone, qui sont des produits de condensation de polysiloxanes substitués par un groupe alcényle et de polysiloxanes ayant des fonctionnalités silane, mélangée(s) avec une ou plusieurs huiles de silicone et facultativement une ou plusieurs huiles adaptées sélectionnées parmi des huiles naturelles, des esters d'acide gras, des monoglycérides, diglycérides et triglycérides, des hydrocarbures cycliques, ramifiés ou linéaires, des alcools gras linéaires ou ramifiés (alcools de Guerbet), et des mélanges de ceux-ci, et la phase aqueuse comporte un ou plusieurs polyols, dans laquelle l'émulsion comprend en outre un émulsifiant, et dans laquelle le polyol est présent dans l'émulsion d'huile dans l'eau en une quantité de 35 à 60 % en poids.

2. Emulsion d'huile dans l'eau selon la revendication 1, dans lequel le polyol est présent dans l'émulsion d'huile dans l'eau en une quantité de 35 à 50 % en poids.

3. Emulsion d'huile dans l'eau selon la revendication 1, dans laquelle le composant huileux comporte :
(a) de 5 à 12 % d'une ou plusieurs cires de silicone ;
(b) de 0,5 à 2,5 % d'une ou plusieurs huiles de silicone ; et
(c) facultativement une ou plusieurs huiles adaptées, et
dans laquelle la phase aqueuse comporte un ou plusieurs polyols, et dans laquelle l'émulsion comporte en outre de 0,3 à 2 % d'un émulsifiant.

4. Emulsion selon la revendication 1 ou 3, dans laquelle le polyol est un polyhydroxyalcane ou un polyhydroxycycloalcane.

5. Emulsion selon la revendication 4, dans laquelle le polyol est de la glycérine.

6. Emulsion selon la revendication 1 ou 3, dans lequel l'émulsifiant est un alcool gras éthoxylé.

7. Emulsion selon la revendication 6, dans lequel l'émulsifiant est sélectionné parmi des alcools gras en C₈₋₁₆ éthoxylés.
